# EUROPEAN PATENT APPLICATION

(11) **EP 4 574 215 A1**
(43) Date of publication of application: **25.06.2025**
(21) Application number: 23383324.3
(22) Date of filing: 20.12.2023
(51) Int. Cl.: A61Q 5/10, A61Q 5/08, A61K 8/22, A61K 8/34, A61K 8/37, A61K 8/92

(54) **OXIDIZING COMPOSITIONS**

(71) Applicant: Beautyge, S.L., 08940 Cornella de Llobregat (ES)
(72) Inventor: BORREDA MORAN, Andrea, 08940 Cornella de Llobregat (Barcelona) (ES); DIAZ POZO, Oscar, 08940 Cornella de Llobregat (Barcelona) (ES); VALLECILLOS LOPEZ, Rocio, 08940 Cornella de Llobregat (Barcelona) (ES)
(74) Representative: Gallardo, Antonio M.

(57) **Abstract**

Oxidizing compositions for oxidatively changing the color of keratin fibers, in particular human hair, are described. Said oxidizing compositions comprise, in a cosmetically acceptable medium, based on the total weight of the compositions: (a) about 0.1-30 wt.% of hydrogen peroxide, (b) about 0.1-50 wt.% of at least one natural oil or fat; and (c) about 0.01-10 wt.% of at least one particular synthetic phenolic antioxidant agent. These oxidizing compositions are stable and can be used in ammonia-free hair dyeing, remaining at least as effective as ammonia compositions in terms of the dyeing power obtained, the chromaticity, the uniformity of dyeing along the fiber, for dyeing processes, and in terms of the lightening and the uniformity of this lightening, for lightening (bleaching) processes.

## Description

### TECHNICAL FIELD

Oxidizing compositions for oxidatively changing the color of keratin fibers, in particular human hair, are described. Said oxidizing compositions comprise, in a cosmetically acceptable medium, based on the total weight of the compositions: (a) about 0.1-30 wt.% of hydrogen peroxide, (b) about 0.1-50 wt.% of at least one natural oil or fat; and (c) about 0.01-10 wt.% of at least one particular synthetic phenolic antioxidant agent. These oxidizing compositions are stable and can be used in ammonia-free hair dyeing, remaining at least as effective as ammonia compositions in terms of the dyeing power obtained, the chromaticity, the uniformity of dyeing along the fiber, for dyeing processes, and in terms of the lightening and the uniformity of this lightening, for lightening (bleaching) processes.

### BACKGROUND

The information provided below is not admitted to be prior art to the present disclosure, but is provided solely to assist the understanding of the reader.

It is known for centuries to apply products to keratinous fibers, for example human hair, either to change or to lighten their color.

To color hair, it can be chosen between three main processes:
1. Lightening (or lifting) the natural color by bleaching the melanin.
2. Adding a new color to supplement the existing hair color by depositing dyes.
3. Do both. Remove or lighten the underlying color and add a second color using dyes.

Most hair color products fall under five major groupings: temporary (dye addition), semi-permanent (dye addition), demi-permanent (dye addition), permanent (lightening and dye addition) and bleach (lightening).

Temporary hair dyeing is usually a leave on product that causes minimal damage to the hair. However, temporary hair dyeing causes stains, and leaches out under rain or with perspiration. Temporary hair dyeing washes out with the next shampoo.

Semi-permanent hair dyeing comes as a rinse, and it causes minimal damage to the hair. However, semi-permanent hair dyeing washes out to some degree with each shampoo and washes out completely within about 4 to 6 shampoos.

Demi-permanent hair dyeing lasts longer than semi-permanent hair dyeing after repeated washing and exposure to sunlight. Both methods are temporary compared to permanent hair color, with demi- lasting up to 30 shampoos.

Demi-permanent contains no ammonia but includes a low-volume developer, containing an oxidizing agent such as, for example, hydrogen peroxide, which allows the dye to penetrate under the outer cuticle of the hair and, in addition, damaging the hair to some extent.

Permanent hair dyeing is characterized by excellent, long-lasting color results. It generally comes in two parts: a dye composition and a developer composition, which contains an oxidizing agent such as, for example, hydrogen peroxide. Usually, permanent hair dyeing compositions do not contain dyestuffs in the conventional sense of the word. They contain colorless precursors which will react with the oxidizing agent inside the hair fiber to produce colored molecules. The dye composition (tint composition) and the developer composition are mixed and then applied to the hair, which is then left for about 10 to about 50 minutes and then rinsed with water.

The oxidizing agent (e.g., hydrogen peroxide) initiates not only the formation of the dyes, but it also breaks down oxidatively the hair's own color pigments (melanins), so a lightened color is simultaneously possible. In order to produce satisfactory coloring and lightening, permanent hair dyeing usually requires an alkaline pH during use; optimal results are achieved in particular at pH values between about 8 and 12.

Typically, all the bleaching methods used in the current techniques are oxidation process. An oxidizing agent (e.g., hydrogen peroxide) is mixed with an alkaline solution and then applied to the hair. The alkaline solution typically contains persulfates (in particular in their sodium, potassium or ammonium salts), sodium percarbonate, perborates (such as sodium perborate or magnesium perborate) and, optionally, peroxides (such as magnesium dioxide or strontium dioxide). As a result, bleaches can deliver a much higher level of lightening than permanent hair dyeing products.

Both monoethanolamine (MEA) and ammonia (NH₃) are used as alkalizing agent in dyeing products. Alkalizing agents serve three important functions: swell the hair fiber to allow better penetration of the dye precursors, generate the active peroxide species necessary for melanin bleaching and dye formation, while also participating in the bleaching of melanin.

Monoethanolamine is increasingly being used for setting and coloring hair and is currently being used as a replacement for ammonia because of ammonia's unpleasant, pungent odor and the possible irritation of the scalp (stinging).

However, one of the key challenges in replacing ammonia with monoethanolamine is the higher percentage of monoethanolamine (in comparison to ammonia) required to deliver the same bleaching effect, commonly referred to as the lightening intensity.

Different ammonia-free permanent hair dyeing products have been proposed with the goal to provide coloring results close to those obtained with ammonia. One option was using a relatively high level of hydrogen peroxide.

Another option was using high levels of oil such as mineral oil either in the dye composition (tint composition), the developer composition (oxidizing composition), in both, or having a third anhydrous composition composed mainly of oil to be mixed with the tint and developer.

Recently, natural oils and, in particular, vegetable oils have been proposed to replace mineral oil. However, the fatty acid alkyl chain of vegetable oils is susceptible to oxidation, resulting in rancid odor and the generation of peroxides species which may interact with other components of the medium (dye precursors in the tint composition or hydrogen peroxide in the developer composition).

It would be desirable to provide the consumer with ammonia-free hair dyeing compositions and methods which may not have these drawbacks, but which may remain at least as effective in terms of the dyeing power obtained, the chromaticity, the uniformity of dyeing along the fiber, for dyeing processes, and in terms of the lightening and the uniformity of this lightening, for lightening (bleaching) processes.

Accordingly, there is a need for improved oxidizing compositions, i.e. hydrogen peroxide based compositions (developer compositions) containing vegetable oils that exhibit long term stability and efficacy.

### SUMMARY

Described herein is an oxidizing composition for oxidatively changing the color of keratin fibers, comprising, in a cosmetically acceptable medium, based on the total weight of the composition:
(a) about 0.1-30 wt.% of hydrogen peroxide;
(b) about 0.1-50 wt.% of at least one natural oil or fat; and
(c) about 0.01-10 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof.

In an embodiment disclosed herein the at least one natural oil or fat is derived from plant (vegetable) materials.

In an embodiment disclosed herein the at least one natural oil or fat is selected from the group consisting of Castor (Ricinus communis) oil, Cocoa (Theobroma cacao) butter, Coconut (Cocus nucifera) oil, Corn (Zea mays) oil, Cotton (Gossypium herbaceum) Seed oil, Linseed (Linum usitatissimum) oil, Olive (Oleo europaea) oil, Palm (Elaies guinensis) oil, Palm-Kernel oil, Peanut (Arachis hypogea) oil, Rapeseed (Brassica napus) oil, Rice (Oryza sativa) Bran oil, Safflower (Carthamus tinctorius) oil, Sesame (Sesamum indicum) oil, Soybean (Glycine max) oil, Sunflower (Helianthus annus) oil, Tall oil, Aceituno (Simarouba glauca) oil, Sweet Almond (Prunus Amygdalus dulcis) oil, Amaranthus (Amaranthus cruentus) oil, Apricot (Prunus armeniaca) seed oil, Avocado (Persea americana) oil, Babassu (Orbignya martiana and O. oleifera) oil, Borage (Borago officinalis) oil, Borneo (Shorea stenoptera) tallow, also known as Illipe butter, Buffalo gourd (Cucurbita foetidissima) oil, Candlenut (Aleurites moluccana) oil, Caraway (Carum carvii) oil, Cashew (Anacardium occidentale) oil, Cherry (Prunus cerasus) oil, Chia (Salvia hispanica) oil, Chinese vegetable tallow and stillingia oil (Sapium sebiferum, Stillingia sebifera), Coriander (Coriandrum sativum) oil, Crambe (Crambe abyssinica, C. hispanica) oil, Cuphea oil, Dimorphotheca (Dimorphotheca pluvialis) seed oil, Camelina (Camelina sativa) oil, Grapeseed (Vitis vinifera) oil, Hazelnut (Corylus avellana) oil, Hemp (Cannabis sativa) seed oil, Honesty (Lunaria annua) oil, Kapok (Bombax malabaricum, Ceiba pentandra) oil, Kokum (Garcinia indica) fat, Lesquerella oil, Macadamia (Macadonia integrifolia, M. tetraphylla) oil, Mango (Mangifer indica) oil, Mango (Mangifer indica) Seed butter, Marigold (Calendula officinalis) oil, Meadowfoam (Limnanthes alba) oil, Melon (Citrullus colocythis and C. vulgaris) oil, Mowrah (Madhuca latifolia, M.longifolia, M.indica) oil, Mustard (Brassica alba, B. hirta, B. nigra, B. juncea, B. carinata) seed oil, Neem (Azadirachta indica) seed oil, Nigella (Nigella sativa, black cumin) oil, Niger (Guizotia abyssinica) oil, Nutmeg (Myristica malabarica and other M. species) oil, Oats (Avena sativa) oil, Oiticica (Licania rigida) Kernel oil, Passionfruit (Passiflora edulis) fruit oil, Perilla (Perilla frutescens) oil, Pistachio (Pistachio vera) oil, Poppy (Papaver somniferium) oil, Purslane (Portulaca oleracea) oil, Sal (Shorea robusta) fat, Sea buckthorn (Hippophae rhamnoides) oil, Shea (Butyrospermum parkii, karite) butter, Tobacco seed oil, Tung (Aleurites fordii) oil, Walnut (Juglans regia) oil, Wheatgerm (Triticum aestivum) oil, and mixtures thereof.

In an embodiment disclosed herein the synthetic phenolic antioxidant is Pentaerythrityl Tetra-Di-t-Butyl Hydroxyhydrocinnamate.

In an embodiment disclosed herein the total amount of (a) hydrogen peroxide is in the range of about 0.5-15 wt.%, based on the total weight of the composition.

In an embodiment disclosed herein the total amount of (b) the at least one natural oil or fat is the range of about 0.5-30 wt.%, based on the total weight of the composition.

In an embodiment disclosed herein the total amount of (c) the at least one synthetic phenolic antioxidant agent is in the range of about 0.1-7.5 wt.%, based on the total weight of the composition.

In an embodiment disclosed herein the oxidizing composition comprises, in a cosmetically acceptable medium, based on the total weight of the composition:
(a) about 0.5-15 wt.% of hydrogen peroxide;
(b) about 0.5-30 wt.% of at least one natural oil or fat; and
(c) about 0.1-7.5 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof.

In an embodiment disclosed herein the oxidizing composition further comprises (d) a non-phenolic antioxidant agent selected from Dilauryl Thiodipropionate.

In an embodiment disclosed herein the oxidizing composition comprises, in a cosmetically acceptable medium, based on the total weight of the composition:
(a) about 0.5-15 wt.% of hydrogen peroxide;
(b) about 0.5-30 wt.% of at least one natural oil or fat;
(c) about 0.1-7.5 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof; and
(d) about 0.1-7.5 wt.% of a non-phenolic antioxidant agent selected from Dilauryl Thiodipropionate.

In an embodiment disclosed herein the keratin fibers are human hair.

In an embodiment disclosed herein the oxidizing composition has a pH in the range from about 1.5 to about 5.5, measured at 25°C.

An embodiment disclosed herein is a process for oxidatively dyeing keratin fibers comprising the steps of:
i) mixing an oxidizing composition as defined above with a tint composition comprising an alkalizing agent and at least one oxidation dye to form a ready-to-use composition;
ii) applying the ready-to-use composition obtained in step (i) to the keratin fibers for a period which is sufficient to obtain the dyeing effect; and
iii) removing the ready-to-use composition from the keratin fibers by rinsing with water.

An embodiment disclosed herein is a process for bleaching or lightening keratin fibers comprising the steps of:
i) mixing an oxidizing composition as above with a bleach composition comprising persulfates, percarbonates, perborates or mixtures thereof to form a ready-to-use composition;
ii) applying the ready-to-use composition obtained in step (i) to the keratin fibers for a period which is sufficient to obtain the bleaching or lightening effect; and
iii) removing the ready-to-use composition from the keratin fibers by rinsing with water.

An embodiment disclosed herein is a multi-compartment device for oxidatively changing the color of keratin fibers comprising at least two compartments packaged separate from one another, wherein
(i) one compartment contains an oxidizing composition comprising, in a cosmetically acceptable medium, based on the total weight of the composition:
   (a) about 0.1-30 wt.% of hydrogen peroxide;
   (b) about 0.1-50 wt.% of at least one natural oil or fat; and
   (c) about 0.01-10 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof; and
(ii) a second compartment contains
   (1) a tint composition comprising an alkalizing agent and at least one oxidation dye; or
   (2) a bleach composition comprising persulfates, percarbonates, perborates or mixtures thereof.

### DETAILED DESCRIPTION

Embodiments of the present disclosure are discussed in detail below. In describing embodiments, specific terminology is employed for the sake of clarity. However, the disclosure is not intended to be limited to the specific terminology so selected. While specific exemplary embodiments are discussed, it should be understood that this is done for illustration purposes only. A person skilled in the relevant art will recognize that other components and configurations can be used without parting from the spirit and scope of the disclosure. While a number of embodiments and features are described herein, it is to be understood that the various features of the disclosure and aspects of embodiments, even if described separately, may be combined unless mutually exclusive or contrary to the specific description. All references cited herein are incorporated by reference as if each had been individually incorporated.

The terms "about" or "approximately" as used herein shall generally mean within 10 percent of a given value. For example, the weight proportion of a compound of about 1% means a weight proportion ranging from 0.9 to 1.1.

Other than in the operating examples, or where otherwise indicated, all numbers expressing quantities of ingredients and/or reaction conditions are to be understood as being modified in all instances by the term "about".

The ranges defined in the specification include the end values as well, i.e. a range of 1 to 10 implies that both 1 and 10 are included in the range. For the avoidance of doubt, the applicant shall be entitled to any equivalents according to applicable law.

The term "water-soluble" as used herein means that at normal temperature and pressure (NTP), defined as 20°C of temperature and 1 atm (101.325 kPa) of pressure.

The term "keratin fibers" as used herein refers to a family of proteins that occur in the vertebrates and exercise a protective function. In an embodiment, keratin fibers refer to human keratin fibers. In another embodiment, keratin fibers refer to human hair.

The term "comprising" refers to optional compatible components/steps that can be used provided that the important ingredients/steps are present.

The term "comprising" thus encompasses and includes the more restrictive terms "consisting of" and "consisting essentially of".

The term "natural oils and fats" refers to oils and fats originating from plant (vegetable) and animal sources. Plant sources include oilseeds, tropical fruits and alga. Animal sources include land-based animals, fish, marine mammals and derived sources. The main constituents of natural oils and fats are linear and/or branched, saturated and/or unsaturated fatty acids with a chain length of 4 up to 22 carbon atoms esterified to glycerol.

The term "synthetic" refers to a compound produced by chemical or biochemical transformation by human intervention.

The term "antioxidant" refers to compounds capable to either delay or inhibit the oxidation process which occur under the influence of atmospheric oxygen or reactive oxygen species.

The term "oxidizing composition" refers to a composition comprising at least one oxidizing agent.

The term "oxidizing agent" refers to an electron accepting compound suitable for use in hair dyeing compositions for removing the natural color of hair (by destroying the melanin pigment) and reacting with oxidative primary dye precursors to enable the reaction with the oxidative coupler dye precursors to form the color dye products. The most commonly used oxidizing agent in the art is hydrogen peroxide, however further suitable oxidizing agents that can be used in combination with hydrogen peroxide are described below. Oxidizing agents also include those which produce hydrogen peroxide when contacted with water. For example, sodium percarbonate, sodium perborate and urea peroxide can be used as anhydrous materials, which when contacted with water release hydrogen peroxide.

The term "oxidation dye" refers to typically colorless materials which will react with an oxidizing agent (typically hydrogen peroxide) inside the hair fiber to produce colored molecules. Oxidation dyes are categorized under two groups: precursors (bases) and couplers. The precursors and peroxide diffuse into the hair shaft, where color formation takes place after a cascade of chemical reactions. The dye precursors are oxidized by hydrogen peroxide to p-benzoquinone imines/diimines, which are reactive intermediates in the color formation. The couplers, which are relatively stable to hydrogen peroxide, undergo rapid reaction with the intermediates resulting in dinuclear, trinuclear or polynuclear colorant molecules. These molecules are too large to escape from the hair structure.

The term "INCI" is the acronym of International Nomenclature Cosmetic Ingredient. INCI names are systematic names internationally recognized to identify cosmetic ingredients. They are developed by the International Nomenclature Committee (INC) and published by the Personal Care Products Council (PCPC) in the *International Cosmetic Ingredient Dictionary and Handbook.*

Typically, the oxidizing composition for oxidatively changing the color of keratin fibers, comprises, in a cosmetically acceptable medium, based on the total weight of the composition:
(a) about 0.1-30 wt.% of hydrogen peroxide;
(b) about 0.1-50 wt.% of at least one natural oil or fat; and
(c) about 0.01-10 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof.

### Cosmetically acceptable medium

The cosmetically acceptable medium for the oxidizing composition is typically an aqueous medium consisting of water and may advantageously contain cosmetically acceptable organic solvents including more particularly alcohols such as ethyl alcohol, isopropyl alcohol, benzyl alcohol and phenylethyl alcohol, glycols or glycol ethers such as, for example, monomethyl, monoethyl and monobutyl ethers of ethylene glycol, propylene glycol or its ethers such as, for example, monomethyl ether of propylene glycol, butylene glycol, dipropylene glycol as well as the alkyl ethers of diethylene glycol such as for example monoethyl ether or monobutyl ether of diethylene glycol. Water may be present in the range of about 30 to about 90 wt. %, about 40 to about 85 wt. %, or about 45 to about 80 wt.%, based on the total weight of the composition. The organic solvents may then be present in concentrations of between about 0.5 and 20 wt. %, or about 2 and 15 wt.%, based on the total weight of the composition.

### Hydrogen peroxide

Hydrogen peroxide [Hydrogen dioxide; CAS RN 7722-84-1] is an odorless and colorless liquid with a bitter taste, and it is slightly more viscous than water, which presents the molecular formula H2O2 and the molecular mass of about 34.0147 g/mol. Hydrogen peroxide has chemical applications, biological function, domestic uses, and therapeutic use, including use as an antimicrobial and oxidizing agent.

The concentration of (a) hydrogen peroxide in the oxidizing composition is determined on the one hand by legal requirements and, on the other hand, by the desired lightening effect.

Typically, in the oxidizing composition, the total amount of (a) hydrogen peroxide (calculated as about 100 percent H2O2), may be in the range of about 0.1 to about 30 wt.%, based on the total weight of the oxidizing composition.

In a particular embodiment, in the oxidizing composition, the total amount of (a) hydrogen peroxide (calculated as about 100 percent H2O2), may be in the range of about 0.5 to about 15 wt.%, about 0.7 to about 12.5 wt.%, about 1 to about 11 wt.%, about 1.5 to about 8 wt.%, or about 2 to about 5 wt.%, based on the total weight of the oxidizing composition.

### The natural oils or fats

As indicated above, the composition for dyeing keratin fibers may comprise (b) at least one natural oil or fat.

In an embodiment, the natural oils or fats originated from plant (vegetable) sources such as oilseeds, tropical fruits and alga.

In another embodiment, the natural oils or fats are selected from the group consisting of Castor (Ricinus communis) oil, Cocoa (Theobroma cacao) butter, Coconut (Cocus nucifera) oil, Corn (Zea mays) oil, Cotton (Gossypium herbaceum) Seed oil, Linseed (Linum usitatissimum) oil, Olive (Oleo europaea) oil, Palm (Elaies guinensis) oil, Palm-Kernel oil, Peanut (Arachis hypogea) oil, Rapeseed (Brassica napus) oil, Rice (Oryza sativa) Bran oil, Safflower (Carthamus tinctorius) oil, Sesame (Sesamum indicum) oil, Soybean (Glycine max) oil, Sunflower (Helianthus annus) oil, Tall oil, Aceituno (Simarouba glauca) oil, Sweet Almond (Prunus Amygdalus dulcis) oil, Amaranthus (Amaranthus cruentus) oil, Apricot (Prunus armeniaca) seed oil, Avocado (Persea americana) oil, Babassu (Orbignya martiana and O. oleifera) oil, Borage (Borago officinalis) oil, Borneo (Shorea stenoptera) tallow, also known as Illipe butter, Buffalo gourd (Cucurbita foetidissima) oil, Candlenut (Aleurites moluccana) oil, Caraway (Carum carvii) oil, Cashew (Anacardium occidentale) oil, Cherry (Prunus cerasus) oil, Chia (Salvia hispanica) oil, Chinese vegetable tallow and stillingia oil (Sapium sebiferum, Stillingia sebifera), Coriander (Coriandrum sativum) oil, Crambe (Crambe abyssinica, C. hispanica) oil, Cuphea oil, Dimorphotheca (Dimorphotheca pluvialis) seed oil, Camelina (Camelina sativa) oil, Grapeseed (Vitis vinifera) oil, Hazelnut (Corylus avellana) oil, Hemp (Cannabis sativa) seed oil, Honesty (Lunaria annua) oil, Kapok (Bombax malabaricum, Ceiba pentandra) oil, Kokum (Garcinia indica) fat, Lesquerella oil, Macadamia (Macadonia integrifolia, M. tetraphylla) oil, Mango (Mangifer indica) oil, Mango (Mangifer indica) Seed butter, Marigold (Calendula officinalis) oil, Meadowfoam (Limnanthes alba) oil, Melon (Citrullus colocythis and C. vulgaris) oil, Mowrah (Madhuca latifolia, M.longifolia, M.indica) oil, Mustard (Brassica alba, B. hirta, B. nigra, B. juncea, B. carinata) seed oil, Neem (Azadirachta indica) seed oil, Nigella (Nigella sativa, black cumin) oil, Niger (Guizotia abyssinica) oil, Nutmeg (Myristica malabarica and other M. species) oil, Oats (Avena sativa) oil, Oiticica (Licania rigida) Kernel oil, Passionfruit (Passiflora edulis) fruit oil, Perilla (Perilla frutescens) oil, Pistachio (Pistachio vera) oil, Poppy (Papaver somniferium) oil, Purslane (Portulaca oleracea) oil, Sal (Shorea robusta) fat, Sea buckthorn (Hippophae rhamnoides) oil, Shea (Butyrospermum parkii, karite) butter, Tobacco seed oil, Tung (Aleurites fordii) oil, Walnut (Juglans regia) oil, Wheatgerm (Triticum aestivum) oil, and mixtures thereof.

In another embodiment, the natural oils or fats are selected from the group consisting of Castor (Ricinus communis) oil, Cocoa (Theobroma cacao) butter, Coconut (Cocus nucifera) oil, Corn (Zea mays) oil, Cotton (Gossypium herbaceum) Seed oil, Linseed (Linum usitatissimum) oil, Olive (Oleo europaea) oil, Palm (Elaies guinensis) oil, Palm-Kernel oil, Peanut (Arachis hypogea) oil, Rapeseed (Brassica napus) oil, Rice (Oryza sativa) Bran oil, Safflower (Carthamus tinctorius) oil, Sesame (Sesamum indicum) oil, Soybean (Glycine max) oil, Sunflower (Helianthus annus) oil, Tall oil, Sweet Almond (Prunus Amygdalus dulcis) oil, Apricot (Prunus armeniaca) seed oil, Avocado (Persea americana) oil, Mango (Mangifer indica) oil, Mango (Mangifer indica) Seed butter, Shea (Butyrospermum parkii, karite) butter, and mixtures thereof.

In a particular embodiment, the natural oils or fats are selected from the group consisting of Cocoa (Theobroma cacao) butter, Coconut (Cocus nucifera) oil, Cotton (Gossypium herbaceum) Seed oil, Olive (Oleo europaea) oil, Soybean (Glycine max) oil, Sunflower (Helianthus annus) oil, Sweet Almond (Prunus Amygdalus dulcis) oil, Avocado (Persea americana) oil, Mango (Mangifer indica) Seed butter, Shea (Butyrospermum parkii, karite) butter, and mixtures thereof.

In an embodiment, the natural oils or fats contain at least one double bound (unsaturation) in the fatty acid alkyl chain.

In another embodiment, the natural oils or fats contain one, two or three double bounds (unsaturation) in the fatty acid alkyl chain.

Typically, in the oxidizing composition, the total amount of (b) the at least one natural oil or fat, may be in the range of about 0.1 to about 50 wt.%, based on the total weight of the oxidizing composition.

In a particular embodiment, in the oxidizing composition, the total amount of (b) the at least one natural oil or fat may be in the range of about 0.5 to about 30 wt.%, about 1 to about 25 wt.%, about 2 to about 20 wt.%, or about 3 to about 18 wt.%, based on the total weight of the oxidizing composition.

### The synthetic phenolic antioxidants

As indicated above, the oxidizing composition may comprise (c) at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof.

Pentaerythrityl Tetra-Di-t-Butyl Hydroxyhydrocinnamate [Pentaerythritol Tetrakis(3-(3,5-di-tert-butyl-4-hydroxyphenyl)propionate), CAS RN 6683-19-8] is a white powder which has a molecular mass of about 1178 g/mol.

Octadecyl Di-t-butyl-4-Hydroxyhydrocinnamate [Octadecyl 3-(3,5-di-tert-butyl-4-hydroxyphenyl)propionate, CAS RN 2082-79-3], may be in the form of white to off-white powder/fine granules and has a molecular mass of about 531 g/mol.

Tetrabutyl Ethylidenebisphenol [2,2'-Ethylidenebis(4,6-di-tert-butylphenol), CAS RN 35958-30-6] may be in the form of a white powder which has a molecular mass of about 438.7 g/mol.

In an embodiment, in the oxidizing composition, the at least one synthetic phenolic antioxidant agent (c) is Pentaerythrityl Tetra-Di-t-Butyl Hydroxyhydrocinnamate.

In another embodiment, in the oxidizing composition, the at least one synthetic phenolic antioxidant agent (c) is Octadecyl Di-t-butyl-4-Hydroxyhydrocinnamate.

In another embodiment, in the oxidizing composition, the at least one synthetic phenolic antioxidant agent (c) is Tetrabutyl Ethylidenebisphenol [2,2'-Ethylidenebis(4,6-di-tert-butylphenol) .

In a particular embodiment, in the oxidizing composition, there is only a synthetic phenolic antioxidant agent (c), which is Pentaerythrityl Tetra-Di-t-Butyl Hydroxyhydrocinnamate.

Typically, in the oxidizing composition, the total amount of (c) at least one synthetic phenolic antioxidant agent, may be in the range of about 0.01 to about 10 wt.%, based on the total weight of the oxidizing composition.

In a particular embodiment, in the oxidizing composition, the total amount of (c) the at least one synthetic phenolic antioxidant agent may be in the range of about 0.1 to about 7.5 wt.%, about 0.2 to about 5 wt.%, about 0.3 to about 3.5 wt.%, or about 0.4 to about 2.5 wt.%, based on the total weight of the oxidizing composition.

### The oxidizing composition

In one embodiment, the oxidizing composition for oxidatively changing the color of keratin fibers, comprises, in a cosmetically acceptable medium, based on the total weight of the composition:
(a) about 0.1-30 wt.% of hydrogen peroxide;
(b) about 0.1-50 wt.% of at least one natural oil or fat; and
(c) about 0.01-10 wt.% of at least one synthetic phenolic antioxidant agent selected from the group consisting of Pentaerythrityl Tetra-Di-t-Butyl, Octadecyl Di-t-butyl-4-Hydroxyhydrocinnamate, Tetrabutyl Ethylidenebisphenol, and mixtures thereof.

In one embodiment, the oxidizing composition for oxidatively changing the color of keratin fibers, comprises, in a cosmetically acceptable medium, based on the total weight of the composition:
(a) about 0.5-15 wt.% of hydrogen peroxide;
(b) about 0.5-30 wt.% of at least one natural oil or fat; and
(c) about 0.1-7.5 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof.

In one embodiment, the oxidizing composition for oxidatively changing the color of keratin fibers, comprises, in a cosmetically acceptable medium, based on the total weight of the composition:
(a) about 0.7-12.5 wt.% of hydrogen peroxide;
(b) about 1-25 wt.% of at least one natural oil or fat; and
(c) about 0.2-5 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof.

In one embodiment, the oxidizing composition for oxidatively changing the color of keratin fibers, comprises, in a cosmetically acceptable medium, based on the total weight of the composition:
(a) about 0.1-30 wt.% of hydrogen peroxide;
(b) about 0.1-50 wt.% of at least one natural oil or fat selected from the group consisting of Castor (Ricinus communis) oil, Cocoa (Theobroma cacao) butter, Coconut (Cocus nucifera) oil, Corn (Zea mays) oil, Cotton (Gossypium herbaceum) Seed oil, Linseed (Linum usitatissimum) oil, Olive (Oleo europaea) oil, Palm (Elaies guinensis) oil, Palm-Kernel oil, Peanut (Arachis hypogea) oil, Rapeseed (Brassica napus) oil, Rice (Oryza sativa) Bran oil, Safflower (Carthamus tinctorius) oil, Sesame (Sesamum indicum) oil, Soybean (Glycine max) oil, Sunflower (Helianthus annus) oil, Tall oil, Aceituno (Simarouba glauca) oil, Sweet Almond (Prunus Amygdalus dulcis) oil, Amaranthus (Amaranthus cruentus) oil, Apricot (Prunus armeniaca) seed oil, Avocado (Persea americana) oil, Babassu (Orbignya martiana and O. oleifera) oil, Borage (Borago officinalis) oil, Borneo (Shorea stenoptera) tallow, also known as Illipe butter, Buffalo gourd (Cucurbita foetidissima) oil, Candlenut (Aleurites moluccana) oil, Caraway (Carum carvii) oil, Cashew (Anacardium occidentale) oil, Cherry (Prunus cerasus) oil, Chia (Salvia hispanica) oil, Chinese vegetable tallow and stillingia oil (Sapium sebiferum, Stillingia sebifera), Coriander (Coriandrum sativum) oil, Crambe (Crambe abyssinica, C. hispanica) oil, Cuphea oil, Dimorphotheca (Dimorphotheca pluvialis) seed oil, Camelina (Camelina sativa) oil, Grapeseed (Vitis vinifera) oil, Hazelnut (Corylus avellana) oil, Hemp (Cannabis sativa) seed oil, Honesty (Lunaria annua) oil, Kapok (Bombax malabaricum, Ceiba pentandra) oil, Kokum (Garcinia indica) fat, Lesquerella oil, Macadamia (Macadonia integrifolia, M. tetraphylla) oil, Mango (Mangifer indica) oil, Mango (Mangifer indica) Seed butter, Marigold (Calendula officinalis) oil, Meadowfoam (Limnanthes alba) oil, Melon (Citrullus colocythis and C. vulgaris) oil, Mowrah (Madhuca latifolia, M.longifolia, M.indica) oil, Mustard (Brassica alba, B. hirta, B. nigra, B. juncea, B. carinata) seed oil, Neem (Azadirachta indica) seed oil, Nigella (Nigella sativa, black cumin) oil, Niger (Guizotia abyssinica) oil, Nutmeg (Myristica malabarica and other M. species) oil, Oats (Avena sativa) oil, Oiticica (Licania rigida) Kernel oil, Passionfruit (Passiflora edulis) fruit oil, Perilla (Perilla frutescens) oil, Pistachio (Pistachio vera) oil, Poppy (Papaver somniferium) oil, Purslane (Portulaca oleracea) oil, Sal (Shorea robusta) fat, Sea buckthorn (Hippophae rhamnoides) oil, Shea (Butyrospermum parkii, karite) butter, Tobacco seed oil, Tung (Aleurites fordii) oil, Walnut (Juglans regia) oil, Wheatgerm (Triticum aestivum) oil, and mixtures thereof; and
(c) about 0.01-10 wt.% of at least one synthetic phenolic antioxidant agent selected from the group consisting of Pentaerythrityl Tetra-Di-t-Butyl , Octadecyl Di-t-butyl-4-Hydroxyhydrocinnamate, Tetrabutyl Ethylidenebisphenol, and mixtures thereof.

In one embodiment, the oxidizing composition for oxidatively changing the color of keratin fibers, comprises, in a cosmetically acceptable medium, based on the total weight of the composition:
(a) about 0.1-30 wt.% of hydrogen peroxide;
(b) about 0.1-50 wt.% of at least one natural oil or fat selected from the group consisting of Castor (Ricinus communis) oil, Cocoa (Theobroma cacao) butter, Coconut (Cocus nucifera) oil, Corn (Zea mays) oil, Cotton (Gossypium herbaceum) Seed oil, Linseed (Linum usitatissimum) oil, Olive (Oleo europaea) oil, Palm (Elaies guinensis) oil, Palm-Kernel oil, Peanut (Arachis hypogea) oil, Rapeseed (Brassica napus) oil, Rice (Oryza sativa) Bran oil, Safflower (Carthamus tinctorius) oil, Sesame (Sesamum indicum) oil, Soybean (Glycine max) oil, Sunflower (Helianthus annus) oil, Tall oil, Sweet Almond (Prunus Amygdalus dulcis) oil, Apricot (Prunus armeniaca) seed oil, Avocado (Persea americana) oil, Mango (Mangifer indica) oil, Mango (Mangifer indica) Seed butter, Shea (Butyrospermum parkii, karite) butter, and mixtures thereof; and
(c) about 0.01-10 wt.% of at least one synthetic phenolic antioxidant agent selected from the group consisting of Pentaerythrityl Tetra-Di-t-Butyl , Octadecyl Di-t-butyl-4-Hydroxyhydrocinnamate, Tetrabutyl Ethylidenebisphenol, and mixtures thereof.

In one embodiment, the oxidizing composition for oxidatively changing the color of keratin fibers, comprises, in a cosmetically acceptable medium, based on the total weight of the composition:
(a) about 0.7-12.5 wt.% of hydrogen peroxide;
(b) about 1-25 wt.% of at least one natural oil or fat selected from the group consisting of Castor (Ricinus communis) oil, Cocoa (Theobroma cacao) butter, Coconut (Cocus nucifera) oil, Corn (Zea mays) oil, Cotton (Gossypium herbaceum) Seed oil, Linseed (Linum usitatissimum) oil, Olive (Oleo europaea) oil, Palm (Elaies guinensis) oil, Palm-Kernel oil, Peanut (Arachis hypogea) oil, Rapeseed (Brassica napus) oil, Rice (Oryza sativa) Bran oil, Safflower (Carthamus tinctorius) oil, Sesame (Sesamum indicum) oil, Soybean (Glycine max) oil, Sunflower (Helianthus annus) oil, Tall oil, Sweet Almond (Prunus Amygdalus dulcis) oil, Apricot (Prunus armeniaca) seed oil, Avocado (Persea americana) oil, Mango (Mangifer indica) oil, Mango (Mangifer indica) Seed butter, Shea (Butyrospermum parkii, karite) butter, and mixtures thereof; and
(c) about 0.2-5 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof.

In one embodiment, the oxidizing composition for oxidatively changing the color of keratin fibers, comprises, in a cosmetically acceptable medium, based on the total weight of the composition:
(a) about 0.7-12.5 wt.% of hydrogen peroxide;
(b) about 1-25 wt.% of at least one natural oil or fat selected from the group consisting of Cocoa (Theobroma cacao) butter, Coconut (Cocus nucifera) oil, Cotton (Gossypium herbaceum) Seed oil, Olive (Oleo europaea) oil, Soybean (Glycine max) oil, Sunflower (Helianthus annus) oil, Sweet Almond (Prunus Amygdalus dulcis) oil, Avocado (Persea americana) oil, Mango (Mangifer indica) Seed butter, Shea (Butyrospermum parkii, karite) butter, and mixtures thereof; and
(c) about 0.2-5 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof.

In one embodiment, the oxidizing composition for oxidatively changing the color of keratin fibers, further comprises (d) a non-phenolic antioxidant agent selected from Dilauryl Thiodipropionate.

Dilauryl Thiodipropionate [Didodecyl 3,3'-thiodipropionate, CAS RN 123-28-4], is typically in the form of white to slightly yellowish flakes, and has a molecular mass of about 515 g/mol.

In an embodiment disclosed herein the oxidizing composition comprises, in a cosmetically acceptable medium, based on the total weight of the composition:
(a) about 0.5-15 wt.% of hydrogen peroxide;
(b) about 0.5-30 wt.% of at least one natural oil or fat;
(c) about 0.1-7.5 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof; and
(d) about 0.1-7.5 wt.% of a non-phenolic antioxidant agent selected from Dilauryl Thiodipropionate.

In an embodiment disclosed herein the oxidizing composition comprises, in a cosmetically acceptable medium, based on the total weight of the composition:
(a) about 0.5-15 wt.% of hydrogen peroxide;
(b) about 0.5-30 wt.% of at least one natural oil or fat;
(c) about 0.1-7.5 wt.% of at least one synthetic phenolic antioxidant agent selected from Pentaerythrityl Tetra-Di-t-Butyl Hydroxyhydrocinnamate; and
(d) about 0.1-7.5 wt.% of a non-phenolic antioxidant agent selected from Dilauryl Thiodipropionate.

In an embodiment disclosed herein the oxidizing composition comprises, in a cosmetically acceptable medium, based on the total weight of the composition:
(a) about 0.5-15 wt.% of hydrogen peroxide;
(b) about 0.5-30 wt.% of at least one natural oil or fat selected from the group consisting of Castor (Ricinus communis) oil, Cocoa (Theobroma cacao) butter, Coconut (Cocus nucifera) oil, Corn (Zea mays) oil, Cotton (Gossypium herbaceum) Seed oil, Linseed (Linum usitatissimum) oil, Olive (Oleo europaea) oil, Palm (Elaies guinensis) oil, Palm-Kernel oil, Peanut (Arachis hypogea) oil, Rapeseed (Brassica napus) oil, Rice (Oryza sativa) Bran oil, Safflower (Carthamus tinctorius) oil, Sesame (Sesamum indicum) oil, Soybean (Glycine max) oil, Sunflower (Helianthus annus) oil, Tall oil, Sweet Almond (Prunus Amygdalus dulcis) oil, Apricot (Prunus armeniaca) seed oil, Avocado (Persea americana) oil, Mango (Mangifer indica) oil, Mango (Mangifer indica) Seed butter, Shea (Butyrospermum parkii, karite) butter, and mixtures thereof;
(c) about 0.1-7.5 wt.% of at least one synthetic phenolic antioxidant agent selected from Pentaerythrityl Tetra-Di-t-Butyl Hydroxyhydrocinnamate; and
(d) about 0.1-7.5 wt.% of a non-phenolic antioxidant agent selected from Dilauryl Thiodipropionate.

In one embodiment, the oxidizing composition for oxidatively changing the color of keratin fibers, comprises, in a cosmetically acceptable medium, based on the total weight of the composition:
(a) about 0.7-12.5 wt.% of hydrogen peroxide;
(b) about 1-25 wt.% of at least one natural oil or fat selected from the group consisting of Cocoa (Theobroma cacao) butter, Coconut (Cocus nucifera) oil, Cotton (Gossypium herbaceum) Seed oil, Olive (Oleo europaea) oil, Soybean (Glycine max) oil, Sunflower (Helianthus annus) oil, Sweet Almond (Prunus Amygdalus dulcis) oil, Avocado (Persea americana) oil, Mango (Mangifer indica) Seed butter, Shea (Butyrospermum parkii, karite) butter, and mixtures thereof; and
(c) about 0.2-5 wt.% of at least one synthetic phenolic antioxidant agent selected from Pentaerythrityl Tetra-Di-t-Butyl Hydroxyhydrocinnamate; and
(d) about 0.2-5 wt.% of a non-phenolic antioxidant agent selected from Dilauryl Thiodipropionate.

In a particular embodiment, the weight ratio between the at least one synthetic phenolic antioxidant agent (c), to the non-phenolic antioxidant agent (d) ranges from about 1:10 to about 10:1, from about 1:5 to about 5:1, from about 1:3 to about 3:1, or from about 1:2 to about 2:1.

In a particular embodiment, the weight ratio between the at least one synthetic phenolic antioxidant agent (c) Pentaerythrityl Tetra-Di-t-Butyl Hydroxyhydrocinnamate, to the non-phenolic antioxidant agent (d) Dilauryl Thiodipropionate ranges from about 1:10 to about 10:1, from about 1:5 to about 5:1, from about 1:3 to about 3:1, or from about 1:2 to about 2:1.

In an embodiment, the oxidizing composition has a pH in the range from about 1.5 to about 5.5, or in the range from about 2 to about 5, measured at 25°C.

In an embodiment, the oxidizing composition is substantially free of any color-imparting compound, i.e. substance capable of imparting a coloration to the keratin fibers, such as pigments, direct-acting dyes, photochromic dyes or thermochromic dyes.

The term "substantially free of any color-imparting compound" refers to an oxidizing composition which contains less than about 0.1 wt.%, less than about 0.01 wt.%, less than about 0.001 wt.% or less than about 0.0001 wt.% of any color-imparting compound, i.e. pigment, direct-acting dye, photochromic dye or thermochromic dye.

In an embodiment, the oxidizing composition is substantially free of any petroleum hydrocarbon, i.e. mineral oil, paraffin and wax obtained from petroleum.

The term "substantially free of any petroleum hydrocarbon" refers to an oxidizing composition which contains less than about 1 wt.%, less than about 0.1 wt.%, less than about 0.01 wt.% or less than about 0.001 wt.% of any petroleum hydrocarbon, i.e. mineral oil, paraffin and wax obtained from petroleum.

In an embodiment, the oxidizing composition is substantially free of hard paraffin, liquid paraffin (Liquid Petrolatum or Paraffinum Liquidum), light liquid paraffin (Light Liquid Petrolatum or Paraffinum Perliquidium), white soft paraffin (White Petrolatum), yellow soft paraffin (Yellow Petrolatum), macrocrystalline paraffin waxes (which are mixtures which consist mainly of saturated C₁₈-C₃₀ hydrocarbons and smaller amounts of iso-alkanes and cycloalkanes with a molecular weight comprised between 250 and 450 g/mol and, although they are solids at room temperature, they have low melting points, usually comprised between 40°C and 60°C), microcrystalline paraffin waxes (which consist of C₄₀-C₅₅ compounds which contain, in addition to normal hydrocarbons, included in the group are iso-alkanes and naphtenes with long alkyl side-chains, the iso-alkanes forming microcrystals, the microcrystalline paraffin waxes having mean molecular weights comprised between 500 and 800 g/mol, being solids at room temperature, and having melting points comprised between 60°C and 90°C), or mixtures thereof.

In an embodiment, the oxidizing composition contains less than about 1 wt.%, less than about 0.1 wt.%, less than about 0.01 wt.% or less than about 0.001 wt.% of hard paraffin, liquid paraffin (Liquid Petrolatum or Paraffinum Liquidum), light liquid paraffin (Light Liquid Petrolatum or Paraffinum Perliquidium), white soft paraffin (White Petrolatum), yellow soft paraffin (Yellow Petrolatum), macrocrystalline paraffin waxes (which are mixtures which consist mainly of saturated C₁₈-C₃₀ hydrocarbons and smaller amounts of iso-alkanes and cycloalkanes with a molecular weight comprised between 250 and 450 g/mol and, although they are solids at room temperature, they have low melting points, usually comprised between 40°C and 60°C), microcrystalline paraffin waxes (which consist of C₄₀-C₅₅ compounds which contain, in addition to normal hydrocarbons, included in the group are iso-alkanes and naphtenes with long alkyl side-chains, the iso-alkanes forming microcrystals, the microcrystalline paraffin waxes having mean molecular weights comprised between 500 and 800 g/mol, being solids at room temperature, and having melting points comprised between 60°C and 90°C), or mixtures thereof.

In an embodiment, the oxidizing composition does not comprise (i.e. is free of) any petroleum hydrocarbon, i.e. mineral oil, paraffin and wax obtained from petroleum.

The term "free of" refers to no detectable amount any petroleum hydrocarbon, i.e. mineral oil, paraffin and wax obtained from petroleum.

In an embodiment, the oxidizing composition does not comprise (i.e. is free of) hard paraffin, liquid paraffin (Liquid Petrolatum or Paraffinum Liquidum), light liquid paraffin (Light Liquid Petrolatum or Paraffinum Perliquidium), white soft paraffin (White Petrolatum), yellow soft paraffin (Yellow Petrolatum), macrocrystalline paraffin waxes (which are mixtures which consist mainly of saturated C₁₈-C₃₀ hydrocarbons and smaller amounts of iso-alkanes and cycloalkanes with a molecular weight comprised between 250 and 450 g/mol and, although they are solids at room temperature, they have low melting points, usually comprised between 40°C and 60°C), microcrystalline paraffin waxes (which consist of C₄₀-C₅₅ compounds which contain, in addition to normal hydrocarbons, included in the group are iso-alkanes and naphtenes with long alkyl side-chains, the iso-alkanes forming microcrystals, the microcrystalline paraffin waxes having mean molecular weights comprised between 500 and 800 g/mol, being solids at room temperature, and having melting points comprised between 60°C and 90°C), or mixtures thereof.

### Other Ingredients

The oxidizing compositions for oxidatively changing the color of keratin fibers may contain a variety of other ingredients to enhance the beneficial properties of the oxidizing compositions, as further described herein.

### 1. Thickening Agents

In an embodiment, the oxidizing compositions may contain one or more agents that will provide a thickening, or viscosity increasing, effect to the oxidizing compositions. Suitable thickening agents include, but are not limited to, anionic, synthetic polymers, cationic synthetic polymers, naturally occurring thickeners, such as nonionic guar gums, scleroglucan gums or xanthan gums, gum arabic, gum ghatti, karaya gum, tragacanth gum, carrageenan gum, agar-agar, locust bean gum, pectins, alginates, starch fractions, and derivatives such as amylose, amylopectin, and dextrins, as well as cellulose derivatives such as, for example, methylcellulose, carboxyalkylcelluloses, and hydroxyalkylcelluloses, nonionic, fully synthetic polymers, such as polyvinyl alcohol or polyvinylpyrrolidinone; as well as inorganic thickeners, in particular phyllosilicates such as, for example, bentonite, in particular smectites, such as montmorillonite or hectorite. Suggested ranges are from about 0.001-20 wt.%, or about 0.005-15 wt.%, or about 0.01-12 wt.%, based on the total weight of the composition.

### 2. Preservatives

In an embodiment, the oxidizing compositions may contain one or more preservatives. Suitable preservatives include, but are not limited to, methyl, ethyl, and propyl paraben, hydantoins, benzoic acid, sodium benzoate, and the like. Suggested ranges are about 0.0001-8 wt.%, about 0.0005-7 wt.%, or about 0.001-5 wt.%, based on the total weight of the composition.

### 3. Chelating Agents

The oxidizing compositions may also contain about 0.0001-5 wt.%, about 0.0005-3 wt.%, or about 0.001-2 wt.%, based on the total weight of the composition, of one or more chelating agents which are capable of complexing with and inactivating metallic ions in order to prevent their adverse effects on the stability or effects of the composition. In particular, the chelating agent will chelate the metal ions found in the water and prevent these ions from interfering with the deposition and reaction of the dye with the hair fiber surface. Suitable chelating agents include, but are not limited to, sodium citrate; nitrogen-containing polycarboxylic acids, particularly Ethylenediaminetetraacetate (EDTA), and calcium, sodium, potassium or triethanolamine derivatives thereof, Hydroxyethyl Ethylenediamine Triacetic Acid (HEDTA), Ethylenediamine-N,N'-disuccinic acid (EDDS); and phosphonates, particularly 1-hydroxyethane-1,1-diphosphonate (HEDP), and/or ethylenediamine tetramethylene phosphonate (EDTMP), and/or diethylenetriamine pentamethylene phosphonate (DTPMP), or sodium salts thereof.

### 4. pH Adjusters

It may also be desirable to add small amounts of acids or buffering systems to adjust the pH of the oxidizing compositions to the desired pH range of about 1.5 to 5.5, measured at 25°C. Suitable acids include hydrochloric acid, phosphoric acid, citric acid, and the like. Suggested ranges of pH adjusters are from about 0.00001-8 wt.%, about 0.00005-6 wt.%, or about 0.0001-5 wt.%, based on the total weight of the composition.

### 5. Stabilizers

The oxidizing compositions may also contain stabilizing agents to prevent any decomposition of the hydrogen peroxide. Sodium metabisulfite (sodium pyrosulfite), sodium sulfite or sodium stannate are typically used for this purpose.

### 6. Nonionic surfactants

Non-limiting examples of nonionic surfactants in the context of the present disclosure include, but are not limited to, alkoxylated C₆-C₂₄ fatty alcohols, polyethoxylated or polypropoxylated fatty acids, alkoxylated triglycerides, alkylphenols, alpha-diols or alcohols having a fatty chain containing, for example, 8 to 18 carbon atoms, ethylene oxide or propylene oxide groups to range in particular from 2 to 50; copolymers of ethylene oxide and of propylene oxide, polyethoxylated fatty amides having from 2 to 30 mol of ethylene oxide; polyglycerolated fatty amides containing on average 1 to 5, or 1.5 to 4, glycerol groups; polyethoxylated fatty amines having 2 to 30 mol of ethylene oxide; oxyethylenated fatty acid esters of sorbitan having from 2 to 30 mol of ethylene oxide; fatty acid esters of sucrose; fatty acid esters of polyethylene glycol; alkylpolyglycosides; N-alkylglucamine derivatives; amine oxides such as (C₁₀-C₁₄) alkylamine oxides or N-acylaminopropylmorpholine oxides.

The term "alkoxylated C₆-C₂₄ fatty alcohol" as used herein refers to ethoxylated or propoxylated C₆-C₂₄ fatty alcohols.

Ethoxylated alcohols (also called polyethyleneglycol ethers or PEG ethers) are produced from the reaction of fatty alcohols with ethylene oxide (EO).

Alkoxylated alcohols (also called polypropyleneglycol ethers or PPG ethers) are produced from the reaction of fatty alcohols with propylene oxide (PO).

Examples of suitable alkoxylated C₆-C₂₄ fatty alcohols include, but are not limited to, ethoxylated palmytil (cetyl) alcohol, ethoxylated palmitoyl alcohol, ethoxylated stearyl alcohol, ethoxylated cetearyl alcohol, ethoxylated isostearyl alcohol, ethoxylated 2-octyldodecanol, ethoxylated 2-ethylhexanoyl alcohol, ethoxylated oleyl alcohol, and mixtures thereof.

Non-limiting examples of alkoxylated C₆-C₂₄ fatty alcohols that may be used according to the present disclosure, include the adducts of ethylene oxide, in particular those containing from 2 to 50 ethylene oxide units, with cetyl alcohol, stearyl alcohol, cetearyl alcohol, isostearyl alcohol, octyldodecanol, 2-ethylhexanoyl alcohol, oleyl alcohol, and mixtures thereof, such as Ceteth-4, Ceteth-5, Ceteth -6, Ceteth-10, Ceteth-12, Ceteth-14, Ceteth-15, Ceteth-16, Ceteth-20, Ceteth-24, Ceteth-25, Ceteth-30, Ceteth-45, Ceteareth-4, Ceteareth-5, Ceteareth-6, Ceteareth-7, Ceteareth-8, Ceteareth-9, Ceteareth-10, Ceteareth-11, Ceteareth-12, Ceteareth-13, Ceteareth-14, Ceteareth-15, Ceteareth-16, Ceteareth-17, Ceteareth-18, Ceteareth-20, Ceteareth-22, Ceteareth-23, Ceteareth-24, Ceteareth-25, Ceteareth-27, Ceteareth-28, Ceteareth-29, Ceteareth-30, Ceteareth-33, Ceteareth-34, Ceteareth-40, Ceteareth-50, Steareth-5, Steareth-8, Steareth-14, Steareth-16, Steareth-21, Steareth-25, Steareth-27, Steareth-30, Steareth-40, Steareth-50, Isosteareth-2, Isoteareth-20, Oleth-2, Oleth-3, Oleth-4, Oleth-5, Oleth-6, Oleth-7, Oleth-8, Oleth-9, Oleth-10, Oleth-11, Oleth-12, Oleth-15, Oleth-16, Oleth-20, Oleth-23, Oleth-25, Oleth-30, Oleth-40, Oleth-44, Oleth-50, and mixtures thereof.

### 7. Anionic surfactants

Non-limiting examples of anionic surfactants include, but are not limited to, alkyl ether carboxylates, alkyl sulfates, alkyl ether sulfates, amide ether sulfates, alkyl glyceride sulfates, olefin sulfonates, alkyl-aryl sulfonates, sulfosuccinates, sulfo fatty acid esters, fatty acid isethionates, fatty acid taurides, phosphate esters, acyl glutamates, acyl peptides, acyl sarcosides, and mixtures thereof. Typically, the at least one anionic surfactant is selected from alkyl sulfates, alkyl ether carboxylates, alkyl ether sulfates, olefin sulfonates, and mixtures thereof.

Alkyl sulfates are obtained by esterification of fatty alcohols (typically having 6 to 24 carbon atoms) with sulfuric acid followed by neutralization. Examples of suitable alkyl sulfates include, but are not limited to, Ammonium Lauryl Sulfate, Sodium Lauryl Sulfate, Potassium Lauryl Sulfate, Ammonium Cetearyl Sulfate, Sodium Cetearyl Sulfate, Potassium Cetearyl Sulfate, Ammonium Stearyl Sulfate, Sodium Stearyl Sulfate and Potassium Stearyl Sulfate.

Alkyl ether carboxylates are obtained by ethoxylation and subsequent carboxymethylation of fatty alcohols (typically having 6 to 24 carbon atoms). Examples of suitable alkyl ether carboxylates include, but are not limited to, Potassium Laureth-4 Carboxylate, Sodium Laureth-4 Carboxylate, Sodium Laureth-5 Carboxylate, Sodium Laureth-6 Carboxylate, and Sodium Laureth-11 Carboxylate.

Alkyl ether sulfates are obtained by the sulfonation of an ethoxylated alcohol. Examples of suitable alkyl ether sulfates include, but are not limited to, Ammonium Laureth Sulfate, Monoethanolamine (MEA)-Laureth Sulfate, Monoisopropanolamine (MIPA)-Laureth Sulfate, Sodium Laureth Sulfate, and Triethanolamine (TEA)-Laureth Sulfate; particularly Sodium Laureth Sulfate having an average degree of ethoxylation of about 1 to 3.

Olefin sulfonates can be produced by sulfonation of an alpha olefin by means of uncomplexed sulfur trioxide, followed by neutralization of the acid reaction mixture in conditions such that any sulfones which have been formed in the reaction are hydrolyzed to give the corresponding hydroxy-alkanesulfonates. Examples of suitable olefin sulfonates include, but are not limited to, C₁₄-C₁₆ Olefin Sulfonate salts, such as Sodium C₁₄-C₁₆ Olefin Sulfonate.

### 8. C₆-C₂₄ fatty alcohols

Non-limiting examples of C₆-C₂₄ fatty alcohols include, but are not limited to C₆-C₂₄ fatty alcohols from vegetable fats and oils and include cotton, safflower, coconut, rapeseed, linseed, palm, palm kernel, sunflower, olein, olive, olive pomace, castor oil, soy, tall oil, etc, possibly totally or partially hydrogenated, as well as purified or synthetic fatty alcohols such as caproyl alcohol, capryl alcohol, capric alcohol, lauryl alcohol, myristyl alcohol, palmytil (cetyl) alcohol, palmitoyl alcohol, stearyl alcohol, isostearyl alcohol, 2-octyldodecanol, 2-ethylhexanoyl alcohol, oleyl alcohol, ricinoleyl alcohol, elaidyl alcohol, petroselinic alcohol, linoleyl alcohol, linolenyl alcohol, arachidyl alcohol, gadoleyl alcohol, behenyl alcohol and erucyl alcohol, or technical grade mixtures.

Examples of suitable C₆-C₂₄ fatty alcohols include, but are not limited to, lauryl alcohol, myristyl alcohol, palmytil (cetyl) alcohol, palmitoyl alcohol, stearyl alcohol, cetearyl alcohol, isostearyl alcohol, 2-octyldodecanol, 2-ethylhexanoyl alcohol, oleyl alcohol, behenyl alcohol, erucyl alcohol, and mixtures thereof, in particular palmytil (cetyl) alcohol, palmitoyl alcohol, stearyl alcohol, cetearyl alcohol, isostearyl alcohol, 2-octyldodecanol, 2-ethylhexanoyl alcohol, oleyl alcohol, behenyl alcohol, erucyl alcohol, and mixtures thereof.

### 9. Silicones

For purposes of the present disclosure silicones include, cyclic and/or linear silicones, which can be found as monomers generally characterized by structural elements such as: 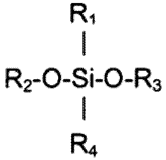 where the silicon atoms can be substituted by alkyl or aryl radicals equal or different, represented here generally by R₁-R₄ groups.

Linear silicones with siloxane units suitable according to the present disclosure are generally characterized by structural elements such as: 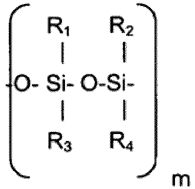 where the silicon atoms can be substituted by alkyl or aryl radicals equal or different, represented here in general by R₁-R₄ groups (meaning the number of different radicals is not necessarily limited to 4), m can take values from 2 to 200.000.

Cyclic silicones suitable according to the present disclosure are generally characterized by structural elements such as: 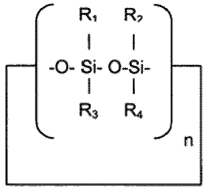 where the silicon atoms can be substituted by alkyl or aryl radicals equal or different, represented here generally by R₁-R₄ groups (meaning the number of different radicals is not necessarily limited to 4), n can take values of 3/2 to 20. Fractional values of n indicate that it may be odd numbers of siloxane groups present in the ring.

Specific examples include a cyclic methyl siloxane having the formula [(CH₃)₂SiO]x in which x is 3-6, or short chain linear methyl siloxanes having the formula ((CH₃)₂SiO[(CH₃)₂SiO]_{y}Si(CH₃)₃ in which y is 0-5.

Some suitable cyclic methyl siloxanes are hexamethylcyclotrisiloxanes (D3), octamethylcyclotetrasiloxane (D4), decamethylcyclopentasiloxane (D5) (cyclomethicone) and dodecamethylcyclohexasiloxane (D6).

Some suitable short linear methyl siloxane are hexamethyldisiloxane (MM), octamethyltrisiloxane (MDM), decamethyltetrasiloxane (MD2M), dodecamethylpentasiloxane (MD3M), tetradecamethylhexasiloxane (MD4M), and hexadecamethylheptasiloxane (MD5M).

Furthermore, long chain linear siloxanes such as phenyltrimethicone, bis(phenylpropyl)dimethicone, dimethicone, dimethiconol, cetyldimethicone and behenoxy dimethicone are also suitable silicones according to the present disclosure.

### 10. Cationic Polymers

The term "cationic polymer" as used herein refers to a macromolecule (polymer) that contains at least one monomer bearing a quaternary ammonium group.

Examples of suitable cationic polymers include, but are not limited to, those polymers known by their International Nomenclature for Cosmetic Ingredients (INCI) name as Polyquaternium. Typical examples of those are Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-42, Polyquaternium-43, Polyquaternium-44, Polyquaternium-45, Polyquaternium-46, Polyquaternium-47, Polyquaternium-48, Polyquaternium-49, Polyquaternium-50, Polyquaternium-51, Polyquaternium-52, Polyquaternium-53, Polyquaternium-54, Polyquaternium-55, Polyquaternium-56, Polyquaternium-57, Polyquaternium-58, Polyquaternium-59, Polyquaternium-60, Polyquaternium-61, Polyquaternium-62, Polyquaternium-63, Polyquaternium-64, Polyquaternium-65, Polyquaternium-66, Polyquaternium-67, Polyquaternium-68, Polyquaternium-69, Polyquaternium-70, Polyquaternium-71, Polyquaternium-72, Polyquaternium-73, Polyquaternium-74, Polyquaternium-75, Polyquaternium-76, Polyquaternium-77, Polyquaternium-78, Polyquaternium-79, Polyquaternium-80, Polyquaternium-81, Polyquaternium-82, Polyquaternium-83, Polyquaternium-84, Polyquaternium-85, Polyquaternium-86, Polyquaternium-87, Polyquaternium-88, Polyquaternium-91, Polyquaternium-92, Polyquaternium-94, Polyquaternium-95, Polyquaternium-96, Polyquaternium-98, Polyquaternium-99, Polyquaternium-100, Polyquaternium-102, Polyquaternium-104, Polyquaternium-109, Polyquaternium-110, Polyquaternium-111, Polyquaternium-112, Polyquaternium-113 and Polyquaternium-114. Other suitable cationic polymers include those known by their INCI name as Guar hydroxypropyl trimonium chloride, Polyacrylamidopropyltrimonium Chloride, Polymethacrylamidopropyltrimonium Chloride and Polymethacrylamidopropyltrimonium Methosulfate.

### 11. Non-ionic polymers

For purposes of this disclosure, non-limiting examples of non-ionic polymers include, but are not limited to:
- nonionic guar gums and modified nonionic guar gums may be for example, but are not limited to, those modified with C₁-C₆ hydroxyalkyl groups;
- biopolysaccharide gums of microbial origin such as scleroglucan gum or xanthan gum;
- gums derived from plant exudates, such as gum arabic, ghatti gum, karaya gum, tragacanth gum, carrageenan, agar and carob gum;
- pectins;
- alginates;
- starches;
- hydroxy (C₁-C₆) alkylcelluloses and carboxy (C₁-C₆) alkylcelluloses;
- celluloses modified with groups comprising at least one fatty chain; non-limiting mention may be made, for example, of hydroxyethylcelluloses modified with groups comprising at least one fatty chain, such as alkyl, arylalkyl or alkylaryl groups, or mixtures thereof, and wherein the alkyl groups are, for example, C₈-C₂₂; or those modified with polyalkylene glycol alkylphenyl ether groups;
- hydroxypropyl guars modified with groups comprising at least one C₈-C₂₂ fatty chain,
- copolymers of vinylpyrrolidone and of hydrophobic monomers comprising a fatty chain, such as vinylpyrrolidone/hexadecene copolymer or vinylpyrrolidone/eicosene copolymer;
- copolymers of C1-C6 alkyl acrylates or methacrylates and of amphiphilic monomers comprising at least one fatty chain;
- copolymers of hydrophilic acrylates or methacrylates and of hydrophobic monomers comprising at least one fatty chain, such as the polyethylene glycol methacrylate/lauryl methacrylate copolymer;
- polymers with an aminoplast ether skeleton comprising at least one fatty chain; and
- polyurethane polyethers comprising in their chain both hydrophilic blocks, for example of polyoxyethylenated nature, and hydrophobic blocks that may be aliphatic blocks alone and/or cycloaliphatic and/or aromatic blocks.

### 12. Amphoteric polymers

For purposes of this disclosure, non-limiting examples of amphoteric polymers include, but are not limited to, amphoteric polysaccharides such as Carboxymethylchitosan or N-[(2'-Hydroxy-2',3'-dicarboxy)ethyl]chitosan; Amphoteric Urethanes; Modified Potato Starch; Methacryloyl Ethyl Betaine/Acrylates Copolymer; Acrylates/Lauryl Acrylate/Stearyl Acrylate/Ethylamine Oxide Methacrylate; and Acrylic acid/(meth)acrylamidopropyl-trimethylarnmonium chloride/stearyl methacrylate terpolymer.

### 13. Anionic polymers

Non-limiting examples of anionic polymers include, but are not limited to, those polymers known by their International Nomenclature for Cosmetic Ingredients (INCI) name as Shellac, Acrylates Copolymer, Acrylates/C1-2 Succinates/Hydroxyacrylates Copolymer, Acrylic Esters (and) Methacrylic Esters Copolymer, AMP-Acrylates/Allyl Methacrylate Copolymer, Diglycol/Cyclohexanedimethanol/Isophtalates/Sulfoisophtalates Copolymer, Diglycol/Isophtalates/Sulfoisophtalates Copolymer, Isobutylene Ethylmaleimide/Hydroxyethylmaleimide Copolymer, Glycerin and Diglycol/Cyclohexanedimethanol/Isophtalates/Sulfoisophtalates Copolymer, Methacrylate Acid/Sodium Acrylamidomethyl Propane Sulfonate Copolymer, Octylacrylamide/Acrylates/Butylaminoethylmethacrylate Copolymer, Polyurethane (and) Acrylates Copolymer, PVM/MA Copolymer, PVP/Ethyl Methacrylate/Methacrylic Acid Terpolymer, PVP/Polycarbamyl Polyglycol Ester, VA/Crotonates Copolymer, VA/Crotonates/Vinyl Neodecanoate Copolymer, VP/Acrylates/Lauryl Methacrylate Copolymer, and their cosmetically acceptable salts.

No particular limitation is imposed on the form of the oxidizing compositions. Examples include transparent liquid, emulsion, cream, mousse, gel, paste, aerosol, and aerosol foam.

Typically, the oxidizing compositions of the present disclosure present a viscosity in the range of about 1,500-70,000 mPa.s, or in the range of about 5,000-50,000 mPa.s, or in the range of about 10,000-40,000 mPa.s, measured at 25°C, using a Brookfield Dial Reading Viscometer LVT with spindle E at 12 rpm.

### The process for dyeing keratin fibers

Another embodiment of the present disclosure refers to a process for oxidatively dyeing keratin fibers comprising the steps of:
i) mixing an oxidizing composition as defined above with a tint composition comprising an alkalizing agent and at least one oxidation dye to form a ready-to-use composition;
ii) applying the ready-to-use composition obtained in step (i) to the keratin fibers for a period which is sufficient to obtain the dyeing effect; and
iii) removing the ready-to-use composition from the keratin fibers by rinsing with water.

In an embodiment disclosed herein is a process for oxidatively dyeing keratin fibers as defined above further comprising the step of topically applying to the keratin fibers a composition comprising at least one conditioning agent, then rinsing with water and optionally followed by washing the keratin fibers with a shampoo, rinsing with water, and optionally drying; or washing the keratin fibers with a shampoo, then rinsing with water and optionally followed by topically applying to the keratin fibers a composition comprising at least one conditioning agent, rinsing with water, and optionally drying.

The term "period which is sufficient to obtain the dyeing effect" refers to the time needed for the hair to develop the desired color. Typically, the ready-to-use composition is allowed to remain in the hair for a period of about 5-60 minutes, or for a period of about 10-50 minutes, at a temperature ranging from about 15-50°C, from about 20-45°C, or from about 20-37°C.

The ready-to-use composition for oxidatively dyeing keratin fibers is typically obtained by mixing a tint composition (composition comprising an alkalizing agent and at least one oxidation dye) and an oxidizing composition (typically a hydrogen peroxide composition) to form a ready-to-use composition, i.e. the dyeing composition which is applied to the keratin fibers.

The mixing ratio of the tint composition and the oxidizing composition may be about 1:1 to 1:4, about 1:1 to 1:3, or about 1:1 to 1:2.5.

It should be noted that the mixing ratio of the oxidizing composition and the tint composition is very much dependent on the level of dyeing effect targeted. The above-mentioned ratios are general and in case somewhat different mixing ratios are needed simply because of reaching higher dyeing level than usual levels, such mixing ratio should still be understood being within the scope of the present disclosure.

Typically, the ready-to-use composition for oxidatively dyeing keratin fibers present a viscosity in the range of about 10,000-70,000 mPa.s, or in the range of about 15,000-60,000 mPa.s, or in the range of about 18,000-55,000 mPa.s, measured at 25°C, using a Brookfield Dial Reading Viscometer LVT with spindle F at 12 rpm.

Typically, the tint composition comprises an alkalizing agent and at least one oxidation dye. The tint composition may contain a variety of other ingredients to enhance the beneficial properties of the dyeing compositions.

The alkalizing agent is typically selected from the group consisting of ammonia (NH₃), monoethanolamine (MEA), 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof.

In a particular embodiment, the alkalizing agent is selected from the group consisting of monoethanolamine (MEA), 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof.

In a particular embodiment, the alkalizing agent is selected from the group consisting of monoethanolamine (MEA), 2-amino-2-methyl-1-propanol (AMP), 2-dimethylamino-2-methyl-1-propanol (DMAMP), and mixtures thereof.

As indicated above, the tint composition may comprise at least one oxidation dye. The oxidation dyes are generally chosen from one or more oxidation precursors (i.e. bases) optionally combined with one or more couplers.

Examples of suitable oxidation precursors include, but are not limited to, p-phenylenediamine, p-toluenediamine, 2-(2-hydroxyethyl)-p-phenylenediamine, 2-(1,2-dihydroxyethyl)-p-phenylenediamine, N,N-bis(2-hydroxyethyl)-p-phenylenediamine, 2-methoxymethyl-p-phenylenediamine, N-(4-amino-3-methylphenyl)-N-[3-(1H-imidazol-1-yl)propyl]amine, N,N'-bis(2-hydroxyethyl)-N,N'-bis(4-aminophenyl)-1,3-diaminopropan-2-ol, bis(2-hydroxy-5-aminophenyl)methane, 1,3-bis(2,5-diaminophenoxy)propan-2-ol, N,N'-bis(4-aminophenyl)-1,4-diazacycloheptane, 1,10-bis(2,5-diaminophenyl)-1,4,7,10-tetraoxadecane, p-aminophenol, 4-amino-3-methylphenol, 4-amino-2-aminomethylphenol, 4-amino-2-(1,2-dihydroxyethyl)phenol, 4-amino-2-(diethylaminomethyl)phenol, 4,5-diamino-1-(2-hydroxyethyl)-1H-pyrazole, 2,4,5,6-tetraaminopyrimidine, 4-hydroxy-2,5,6-triaminopyrimidine, 2-hydroxy-4,5,6-triaminopyrimidine, 2,3-diamino-6,7-dihydro-1H,5H-pyrazolo[1,2-a]pyrazol-1-one, and the physiologically acceptable salts thereof.

Examples of suitable couplers include, but are not limited to, 3-aminophenol, 5-amino-2-methylphenol, 3-amino-2-chloro-6-methylphenol, 2-hydroxy-4-aminophenoxyethanol, 5-amino-4-chloro-2-methylphenol, 5-(2-hydroxyethyl)amino-2-methylphenol, 2,4-dichloro-3-aminophenol, 2-aminophenol, 3-phenylenediamine, 2-(2,4-diaminophenoxy)ethanol, 1,3-bis(2,4-diaminophenoxy)propane, 1-methoxy-2-amino-4-(2-hydroxyethylamino)benzene, 1,3-bis(2,4-diaminophenyl)propane, 2,6-bis(2'-hydroxyethylamino)-1-methylbenzene, 2-({3-[(2-hydroxyethyl)amino]-4-methoxy-5-methylphenyl}amino)ethanol, 2-({3-[(2-hydroxyethyl)amino]-2-methoxy-5-methylphenyl}-amino)ethanol, 2-({3-[(2-hydroxyethyl)amino]-4,5-dimethylphenyl}amino)ethanol, 2-[3-morpholin-4-ylphenyl)amino]ethanol, 3-amino-4-(2-methoxyethoxy)-5-methylphenylamine, 1-amino-3-bis(2-hydroxyethyl)aminobenzene, resorcinol, 2-methylresorcinol, 4-chlororesorcinol, 1,2,4-trihydroxybenzene, 2-amino-3-hydroxypyridine, 3-amino-2-methylamino-6-methoxypyridine, 2,6-dihydroxy-3,4-dimethylpyridine, 3,5-diamino-2,6-dimethoxypyridine, 1-phenyl-3-methylpyrazol-5-one, 1-naphthol, 1,5-dihydroxynaphthalene, 2,7-dihydroxynaphthalene, 1,7-dihydroxynaphthalene, 1,8-dihydroxynaphthalene, 4-hydroxyindole, 6-hydroxyindole, 7-hydroxyindole, 4-hydroxyindoline, 6-hydroxyindoline, 7-hydroxyindoline, or mixtures of said compounds or the physiologically acceptable salts thereof.

Another embodiment of the present disclosure refers to a process for bleaching or lightening keratin fibers comprising the steps of:
i) mixing an oxidizing composition as defined above with a bleach composition comprising persulfates, percarbonates, perborates or mixtures thereof to form a ready-to-use composition;
ii) applying the ready-to-use composition obtained in step (i) to the keratin fibers for a period which is sufficient to obtain the bleaching or lightening effect; and
iii) removing the ready-to-use composition from the keratin fibers by bleaching or lightening effect rinsing with water.

In an embodiment disclosed herein is a process for bleaching or lightening keratin fibers as defined above further comprising the step of topically applying to the keratin fibers a composition comprising at least one conditioning agent, then rinsing with water and optionally followed by washing the keratin fibers with a shampoo, rinsing with water, and optionally drying; or washing the keratin fibers with a shampoo, then rinsing with water and optionally followed by topically applying to the keratin fibers a composition comprising at least one conditioning agent, rinsing with water, and optionally drying.

The term "period which is sufficient to obtain the bleaching or lightening effect" refers to the time needed for the hair to develop the desired color. Typically, the ready-to-use composition is allowed to remain in the hair for a period of about 5-75 minutes, or for a period of about 10-45 minutes, at a temperature ranging from about 15-50°C, from about 20-45°C, or from about 20-37°C.

Typically, the bleach composition is in the form of an anhydrous composition. The term "anhydrous" as used herein refers to a composition whose water content is less than 1 % by weight, relative to the total weight of the bleach composition.

Typically, the bleach composition may contain a variety of other ingredients to enhance the beneficial properties of said bleach compositions such as anionic, nonionic, cationic, amphoteric or zwitterionic surfactants and mixtures thereof, petroleum hydrocarbons (mineral oils, paraffins and waxes), vegetable fats and oils, fatty acids, fatty alcohols, natural waxes, silicones, granulating adjuvants, binders, mineral fillers, lubricants, opacifiers, inorganic pigments, dyes, sequestering agents, fragrances or perfumes and nonionic or amphoteric polymers.

Application of the ready-to-use composition either for dyeing keratin fibers or for bleaching or lightening keratin fibers may be undertaken in several ways. Application of the ready-to-use composition may take place on the whole head of hair of an end user.

As used herein, the "whole head of hair" means that the hair all over the head from the root of the hair to the tip of the hair is included in the application process.

By contrast, the application of the ready-to-use composition may take place only on the root portion of the hair. The application to the root portion of the hair may still be over the entire head of the end user, but application of the ready-to-use composition is applied only to the section of hair closest to the head (root portion), which is between about 0.01 mm to about 40 mm from the scalp of the head. After application to the root portion, the ready-to-use composition may be applied to the rest of the hair at a later stage to prevent over processing of the hair in the lengths and ends.

Also, application may take place on a portion of hair. Application of a portion of hair is commonly referred to as highlighting or lowlighting. The portion of hair may be physically separated from the whole head of hair in a hair bundle or may be a smaller portion of hair than the whole head of hair. A hair bundle may be physically separated from a whole head of hair by a device including a plastic cap through which hair bundles are formed when hair is pulled through orifices in the plastic cap, metal foils encompassing a hair bundle, strand separators applied to hair at the root portion, or similar devices.

The application of the ready-to-use composition may be done by hand or by a tool for a period which is sufficient to obtain the dyeing effect to ensure uniform application to the entire treated hair surface.

The ready-to-use composition can be applied on hair via an applicator bottle or brush. It can be used on full head or partly on single strands (highlight application) as common highlight applicator foils, caps and special applicators can be used, but also freehand techniques such as balayage, with brush and/or combs can be possible. The composition can also be applied as a mousse via a manual spray, a pressurized container or an aerosol mousse.

### The multi-compartment device

Another embodiment of the present disclosure refers to a multi-compartment device for oxidatively changing the color of keratin fibers comprising at least two compartments packaged separate from one another, wherein
(i) one compartment contains an oxidizing composition comprising, in a cosmetically acceptable medium, based on the total weight of the composition:
   (a) about 0.1-30 wt.% of hydrogen peroxide;
   (b) about 0.1-50 wt.% of at least one natural oil or fat; and
   (c) about 0.01-10 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof; and
(ii) a second compartment contains
   (1) a tint composition comprising an alkalizing agent and at least one oxidation dye; or
   (2) a bleach composition comprising persulfates, percarbonates, perborates or mixtures thereof.

In another embodiment, the multi-compartment device for oxidatively changing the color of keratin fibers comprising at least two compartments packaged separate from one another, wherein
(i) one compartment contains an oxidizing composition comprising, in a cosmetically acceptable medium, based on the total weight of the composition:
   (a) about 0.1-30 wt.% of hydrogen peroxide;
   (b) about 0.1-50 wt.% of at least one natural oil or fat; and
   (c) about 0.01-10 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof; and
(ii) a second compartment contains a tint composition comprising an alkalizing agent and at least one oxidation dye.

In another embodiment, the multi-compartment device for dyeing keratin fibers comprises at least two compartments packaged separate from one another, wherein:
(i) one compartment contains an oxidizing composition comprising, in a cosmetically acceptable medium, based on the total weight of the composition:
   (a) about 0.5-15 wt.% of hydrogen peroxide;
   (b) about 0.5-30 wt.% of at least one natural oil or fat;
   (c) about 0.1-7.5 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof; and
(ii) a second compartment contains a tint composition comprising an alkalizing agent and at least one oxidation dye.

In another embodiment, the multi-compartment device for dyeing keratin fibers comprises at least two compartments packaged separate from one another, wherein:
(i) one compartment contains an oxidizing composition comprising, in a cosmetically acceptable medium, based on the total weight of the composition:
   (a) about 0.5-15 wt.% of hydrogen peroxide;
   (b) about 0.5-30 wt.% of at least one natural oil or fat;
   (c) about 0.1-7.5 wt.% of at least one synthetic phenolic antioxidant agent selected from Pentaerythrityl Tetra-Di-t-Butyl Hydroxyhydrocinnamate; and
(ii) a second compartment contains a tint composition comprising an alkalizing agent and at least one oxidation dye.

In another embodiment, the multi-compartment device for dyeing keratin fibers comprises at least two compartments packaged separate from one another, wherein:
(i) one compartment contains an oxidizing composition comprising, in a cosmetically acceptable medium, based on the total weight of the composition:
   (a) about 0.5-15 wt.% of hydrogen peroxide;
   (b) about 0.5-30 wt.% of at least one natural oil or fat;
   (c) about 0.1-7.5 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof; and
   (d) about 0.1-7.5 wt.% of a non-phenolic antioxidant agent selected from Dilauryl Thiodipropionate; and
(ii) a second compartment contains a tint composition comprising an alkalizing agent and at least one oxidation dye.

In another embodiment, the multi-compartment device for dyeing keratin fibers comprises at least two compartments packaged separate from one another, wherein:
(i) one compartment contains an oxidizing composition comprising, in a cosmetically acceptable medium, based on the total weight of the composition:
   (a) about 0.5-15 wt.% of hydrogen peroxide;
   (b) about 0.5-30 wt.% of at least one natural oil or fat;
   (c) about 0.1-7.5 wt.% of at least one synthetic phenolic antioxidant agent selected from Pentaerythrityl Tetra-Di-t-Butyl Hydroxyhydrocinnamate; and
   (d) about 0.1-7.5 wt.% of a non-phenolic antioxidant agent selected from Dilauryl Thiodipropionate; and
(ii) a second compartment contains a tint composition comprising an alkalizing agent and at least one oxidation dye.

In another embodiment, the multi-compartment device for oxidatively changing the color of keratin fibers comprises at least three compartments packaged separate from one another, wherein:
(i) one compartment contains an oxidizing composition comprising, in a cosmetically acceptable medium, based on the total weight of the composition:
   (a) about 0.1-30 wt.% of hydrogen peroxide;
   (b) about 0.1-50 wt.% of at least one natural oil or fat; and
   (c) about 0.01-10 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof; and
(ii) a second compartment contains a tint composition comprising an alkalizing agent and at least one oxidation dye; and
(iii) a third compartment contains a bleach composition comprising persulfates, percarbonates, perborates or mixtures thereof.

In another embodiment, the multi-compartment device for oxidatively changing the color of keratin fibers comprises at least three compartments packaged separate from one another, wherein:
(i) one compartment contains an oxidizing composition comprising, in a cosmetically acceptable medium, based on the total weight of the composition:
   (a) about 0.1-30 wt.% of hydrogen peroxide;
   (b) about 0.1-50 wt.% of at least one natural oil or fat; and
   (c) about 0.01-10 wt.% of at least one synthetic phenolic antioxidant agent selected from Pentaerythrityl Tetra-Di-t-Butyl Hydroxyhydrocinnamate; and
(ii) a second compartment contains a tint composition comprising an alkalizing agent and at least one oxidation dye; and
(iii) a third compartment contains a bleach composition comprising persulfates, percarbonates, perborates or mixtures thereof.

In another embodiment, the multi-compartment device for oxidatively changing the color of keratin fibers comprises at least three compartments packaged separate from one another, wherein:
(i) one compartment contains an oxidizing composition comprising, in a cosmetically acceptable medium, based on the total weight of the composition:
   (a) about 0.1-30 wt.% of hydrogen peroxide;
   (b) about 0.1-50 wt.% of at least one natural oil or fat; and
   (c) about 0.01-10 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof; and
   (d) about 0.1-7.5 wt.% of a non-phenolic antioxidant agent selected from Dilauryl Thiodipropionate; and
(ii) a second compartment contains a tint composition comprising an alkalizing agent and at least one oxidation dye; and
(iii) a third compartment contains a bleach composition comprising persulfates, percarbonates, perborates or mixtures thereof.

In another embodiment, the multi-compartment device for oxidatively changing the color of keratin fibers comprises at least three compartments packaged separate from one another, wherein:
(i) one compartment contains an oxidizing composition comprising, in a cosmetically acceptable medium, based on the total weight of the composition:
   (a) about 0.1-30 wt.% of hydrogen peroxide;
   (b) about 0.1-50 wt.% of at least one natural oil or fat; and
   (c) about 0.01-10 wt.% of at least one synthetic phenolic antioxidant agent selected from Pentaerythrityl Tetra-Di-t-Butyl Hydroxyhydrocinnamate; and
   (d) about 0.1-7.5 wt.% of a non-phenolic antioxidant agent selected from Dilauryl Thiodipropionate; and
(ii) a second compartment contains a tint composition comprising an alkalizing agent and at least one oxidation dye; and
(iii) a third compartment contains a bleach composition comprising persulfates, percarbonates, perborates or mixtures thereof.

Typically, the multi-compartment device for oxidatively changing the color of keratin fibers is a multi-component packaging unit (kit-of-parts) including at least two compartments packaged separate from one another, wherein one compartment contains an oxidizing composition as defined above; and a second compartment contains a tint composition comprising an alkalizing agent and at least one oxidation dye.

The oxidizing composition as defined above and the tint composition, packaged separate from one another, can be provided in separate containers, which are located together in an outer package (i.e. a cardboard box or a carton). Alternatively, the oxidizing composition as defined above and the tint composition can be provided in two separate containers, which are purchased separately from one another and are mixed together before use.

The oxidizing composition as defined above and the tint composition, packaged separate from one another, can also be provided in a single multi-chamber container (dispenser) such as an aerosol can.

When present, an optional conditioning agent can be provided in an additional container. In the latter case, the conditioner can be mixed immediately before use and applied together with the other components, or the content of the additional container can be applied (after an optional rinse step) as a post-treatment immediately after the composition for dyeing keratin fibers.

The following examples are given in order to provide a person skilled in the art with a sufficiently clear and complete explanation of the present disclosure but should not be considered as limiting of the essential aspects of its subject, as set out in the preceding portions of this description.

### EXAMPLES

### 1. Preparation of the oxidizing compositions

The compositions of Table 1 were prepared. CE1 represents a Comparative Experiment.

**Table 1. - Oxidizing compositions (wt. % based on the total weight of the composition).**

| **Ingredients (INCI)** | **C1** | **CE1** |
|---|---|---|
| Coconut Oil | 10 | 10 |
| Sunflower Oil | 5 | 5 |
| Pentaerythrityl Tetra-Di-t-Butyl Hydroxyhydrocinnamate | 0.5 | --- |
| Hydrogen Peroxide (as 100 H₂O₂) | 10.5 | 10.5 |
| Cetearyl Alcohol | 5 | 5 |
| PEG-40 Castor Oil | 2 | 2 |
| Sodium Cetearyl Sulfate | 1 | 1 |
| Aqua (Water) (Eau), preservatives, chelating agents, pH adjusters and stabilizers | Up to 100 | Up to 100 |

### 2. Determination of the stability of the oxidizing compositions

### 2.1. Fatty Acid Methyl Ester (FAME) analysis

The oxidizing compositions described in Table 1 were stored at room temperature (RT; about 25°C) and about 45°C for a period of up to 12 weeks.

The stability of the compositions, i.e. stability of the oils, was evaluated by Fatty Acid Methyl Ester (FAME) analysis. When the oxidation of the fatty acid alkyl chain takes place, the content of fatty acids with double bonds (such as oleic acid or linoleic acid), and as a consequence its methyl esters, decreased over time.

A drying process, followed by a methylation by heating with sodium methylate in methanol, followed by heating in acidic medium was carried out as follows:
Approximately 3 g of the oxidizing compositions described in Table 1 were transferred into a glass crucible and dried in a non-convection oven for 2 hours at 105°C. 0.25-0.30 g of the dry matter of the oxidizing compositions described in Table 1 were transferred into a 100 mL ground-necked volumetric flask. With the aid of a test tube, 12 mL of a 0.2 N sodium methylate in methanol solution and boiling chips were added. A reflux condenser was fitted, and the solution was stirred and brought to the boil. The solution should become clear, which usually occurs in about 10 minutes. The reaction is complete after 15 minutes.

The flask was taken away from heating and, when the reflux stopped, the condenser was removed. The flask was allowed to cool until room temperature and two drops of a 1% phenolphthalein solution in methanol were added. The solution became pink. 17 mL of a 1N sulphuric acid in methanol solution were added until the solution became colorless.

The reflux condenser was fitted again, and the solution was stirred and brought to the boil for 5-10 minutes.

The source of heat was withdrawn, and the flask was cooled under running water. The reflux condenser was removed, and 20 mL of a saturated sodium chloride aqueous solution were added under stirring. 8 mL of heptane were added, and the flask was plugged and shacked vigorously for 15 seconds. The flask was left to settle until the two phases were separated. 20 mL of the saturated sodium chloride aqueous solution were added again until the aqueous layer reached the lower end of the flask neck. The upper layer containing the methyl esters filled the flask neck. Finally, with the help of a Pasteur pipette, a portion of the upper layer was transferred to a GC vial for analysis. This process was repeated three times for each oxidizing composition.

Analysis of FAMEs was performed with the HP 6890N Gas Chromatograph from Agilent Technologies equipped with an integrated liquid autosampler, capillary injector and FID Detector. The configuration and method parameters are listed in Table 2 and Table 3 below.

**Table 2 - Gas Chromatograph configuration**

| | |
|---|---|
| Gas Chromatograph | HP 6890N Agilent Technologies |
| Injector | Split/Splitless Injector |
| Detector | Flame Ionization Detector |
| Software | Agilent ChemStation |
| Column | Supelco SP-2560 Fused Silica Capillary Column, L × I.D. 100 m × 0.25 mm, df 0.20 µm |

**Table 3 - Gas Chromatograph method parameters**

| Injector Parameters | |
|---|---|
| Injection Volume | 1 µL |
| Type | Split |
| Temperature | 260°C |
| Split | 1:25 |

| Detector | |
|---|---|
| Type | FID |
| Temperature | 280°C |
| H₂ flow rate | 35 mL/min |

| Oven Parameters | |
|---|---|
| Initial Temperature | 180°C |
| Hold | 47 min |
| Ram p Rate | 20°C/min |
| Temperature | 230°C |
| Hold | 20 min |
| Total Runtime | 69.50 min |

### 2.2. Results

The ratio of all identified fatty acids was calculated using the integrated peak areas of the FAMEs identified in the samples.

The mean value of the three replicates of the analysis, each one corresponding to different drying and methylation processes, per each oxidizing composition, was calculated. The results of the fatty acids of interest, oleic acid and linoleic acid, are listed in Table 4.

**Table 4 - Total oleic acid (C18:1) and linoleic acid (C18:2) content (wt.%)**

| | **C1 12 weeks at RT** | **C1 12 weeks at 45°C** | **CE1 12 weeks at RT** | **CE1 12 weeks at 45°C** |
|---|---|---|---|---|
| Methylation 1 | 27.03 | 25.78 | 25.83 | 18.18 |
| Methylation 2 | 26.90 | 25.52 | 26.56 | 18.84 |
| Methylation 2 | 27.34 | 25.36 | 25.84 | 16.81 |
| **Mean** | 27.09 | 25.55 | 26.08 | 17.94 |
| **Standard deviation** | 0.23 | 0.21 | 0.42 | 1.04 |
| **Reduction in the content of oleic acid and linoleic acid** | 5.7% | | 31.2% | |

From the experimental results, it can be concluded that the oxidizing compositions of the disclosure present were more stable (less reduction in the content of unsaturated fatty acids) than Comparative Experiment CE1.

Modifications, which do not affect, alter, change or modify the essential aspects of the compositions and methods described above, are included within the scope of the present disclosure.

## Claims

1. An oxidizing composition for oxidatively changing the color of keratin fibers, comprising, in a cosmetically acceptable medium, based on the total weight of the composition:
(a) about 0.1-30 wt.% of hydrogen peroxide;
(b) about 0.1-50 wt.% of at least one natural oil or fat; and
(c) about 0.01-10 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof.

2. The oxidizing composition according to claim 1, wherein the at least one natural oil or fat is derived from plant (vegetable) materials.

3. The oxidizing composition according to anyone of claims 1 to 2, wherein the at least one natural oil or fat is selected from the group consisting of Castor (Ricinus communis) oil, Cocoa (Theobroma cacao) butter, Coconut (Cocus nucifera) oil, Corn (Zea mays) oil, Cotton (Gossypium herbaceum) Seed oil, Linseed (Linum usitatissimum) oil, Olive (Oleo europaea) oil, Palm (Elaies guinensis) oil, Palm-Kernel oil, Peanut (Arachis hypogea) oil, Rapeseed (Brassica napus) oil, Rice (Oryza sativa) Bran oil, Safflower (Carthamus tinctorius) oil, Sesame (Sesamum indicum) oil, Soybean (Glycine max) oil, Sunflower (Helianthus annus) oil, Tall oil, Aceituno (Simarouba glauca) oil, Sweet Almond (Prunus Amygdalus dulcis) oil, Amaranthus (Amaranthus cruentus) oil, Apricot (Prunus armeniaca) seed oil, Avocado (Persea americana) oil, Babassu (Orbignya martiana and O. oleifera) oil, Borage (Borago officinalis) oil, Borneo (Shorea stenoptera) tallow, also known as Illipe butter, Buffalo gourd (Cucurbita foetidissima) oil, Candlenut (Aleurites moluccana) oil, Caraway (Carum carvii) oil, Cashew (Anacardium occidentale) oil, Cherry (Prunus cerasus) oil, Chia (Salvia hispanica) oil, Chinese vegetable tallow and stillingia oil (Sapium sebiferum, Stillingia sebifera), Coriander (Coriandrum sativum) oil, Crambe (Crambe abyssinica, C. hispanica) oil, Cuphea oil, Dimorphotheca (Dimorphotheca pluvialis) seed oil, Camelina (Camelina sativa) oil, Grapeseed (Vitis vinifera) oil, Hazelnut (Corylus avellana) oil, Hemp (Cannabis sativa) seed oil, Honesty (Lunaria annua) oil, Kapok (Bombax malabaricum, Ceiba pentandra) oil, Kokum (Garcinia indica) fat, Lesquerella oil, Macadamia (Macadonia integrifolia, M. tetraphylla) oil, Mango (Mangifer indica) oil, Mango (Mangifer indica) Seed butter, Marigold (Calendula officinalis) oil, Meadowfoam (Limnanthes alba) oil, Melon (Citrullus colocythis and C. vulgaris) oil, Mowrah (Madhuca latifolia, M.longifolia, M.indica) oil, Mustard (Brassica alba, B. hirta, B. nigra, B. juncea, B. carinata) seed oil, Neem (Azadirachta indica) seed oil, Nigella (Nigella sativa, black cumin) oil, Niger (Guizotia abyssinica) oil, Nutmeg (Myristica malabarica and other M. species) oil, Oats (Avena sativa) oil, Oiticica (Licania rigida) Kernel oil, Passionfruit (Passiflora edulis) fruit oil, Perilla (Perilla frutescens) oil, Pistachio (Pistachio vera) oil, Poppy (Papaver somniferium) oil, Purslane (Portulaca oleracea) oil, Sal (Shorea robusta) fat, Sea buckthorn (Hippophae rhamnoides) oil, Shea (Butyrospermum parkii, karite) butter, Tobacco seed oil, Tung (Aleurites fordii) oil, Walnut (Juglans regia) oil, Wheatgerm (Triticum aestivum) oil, and mixtures thereof.

4. The oxidizing composition according to anyone of claims 1 to 4, wherein the synthetic phenolic antioxidant is Pentaerythrityl Tetra-Di-t-Butyl Hydroxyhydrocinnamate.

5. The oxidizing composition according to anyone of claims 1 to 5, wherein the total amount of (a) hydrogen peroxide is in the range of about 0.5-15 wt.%, based on the total weight of the composition.

6. The oxidizing composition according to anyone of claims 1 to 6, wherein the total amount of (b) the at least one natural oil or fat is the range of about 0.5-30 wt.%, based on the total weight of the composition.

7. The oxidizing composition according to anyone of claims 1 to 7, wherein the total amount of (c) the at least one synthetic phenolic antioxidant agent is in the range of about 0.1-7.5 wt.%, based on the total weight of the composition.

8. The oxidizing composition according to anyone of claims 1 to 8 comprising, in a cosmetically acceptable medium, based on the total weight of the composition:
(a) about 0.5-15 wt.% of hydrogen peroxide;
(b) about 0.5-30 wt.% of at least one natural oil or fat; and
(c) about 0.1-7.5 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof.

9. The oxidizing composition according to anyone of claims 1 to 8, further comprising (d) a non-phenolic antioxidant agent selected from Dilauryl Thiodipropionate.

10. The oxidizing composition according to claim 9, comprising, in a cosmetically acceptable medium, based on the total weight of the composition:
(a) about 0.5-15 wt.% of hydrogen peroxide;
(b) about 0.5-30 wt.% of at least one natural oil or fat;
(c) about 0.1-7.5 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof; and
(d) about 0.1-7.5 wt.% of a non-phenolic antioxidant agent selected from Dilauryl Thiodipropionate.

11. The oxidizing composition according to anyone of claims 1 to 10, wherein the keratin fibers are human hair.

12. The oxidizing composition according to anyone of claims 1 to 12, wherein the oxidizing composition has a pH in the range from about 1.5 to about 5.5, measured at 25°C.

13. A process for oxidatively dyeing keratin fibers comprising the steps of:
i) mixing an oxidizing composition as defined in any of claims 1 to 12 with a tint composition comprising an alkalizing agent and at least one oxidation dye to form a ready-to-use composition;
ii) applying the ready-to-use composition obtained in step (i) to the keratin fibers for a period which is sufficient to obtain the dyeing effect; and
iii) removing the ready-to-use composition from the keratin fibers by rinsing with water.

14. A process for bleaching or lightening keratin fibers comprising the steps of:
i) mixing an oxidizing composition as defined in any of claims 1 to 12 with a bleach composition comprising persulfates, percarbonates, perborates or mixtures thereof to form a ready-to-use composition;
ii) applying the ready-to-use composition obtained in step (i) to the keratin fibers for a period which is sufficient to obtain the bleaching or lightening effect; and
iii) removing the ready-to-use composition from the keratin fibers by rinsing with water.

15. A multi-compartment device for oxidatively changing the color of keratin fibers comprising at least two compartments packaged separate from one another, wherein
(i) one compartment contains an oxidizing composition comprising, in a cosmetically acceptable medium, based on the total weight of the composition:
(a) about 0.1-30 wt.% of hydrogen peroxide;
(b) about 0.1-50 wt.% of at least one natural oil or fat; and
(c) about 0.01-10 wt.% of at least one synthetic phenolic antioxidant agent selected from the group consisting of Pentaerythrityl Tetra-Di-t-Butyl Hydroxyhydrocinnamate, Octadecyl Di-t-butyl-4-Hydroxyhydrocinnamate, Tetrabutyl Ethylidenebisphenol, and mixtures thereof; and
(ii) a second compartment contains
(1) a tint composition comprising an alkalizing agent and at least one oxidation dye; or
(2) a bleach composition comprising persulfates, percarbonates, perborates or mixtures thereof.
